# EUROPEAN PATENT APPLICATION

(11) **EP 2 549 397 A1**
(43) Date of publication of application: **23.01.2013**
(21) Application number: 12174584.8
(22) Date of filing: 02.07.2012
(51) Int. Cl.: G06F 19/00

(54) **Method for customizing a hearing aid**

(71) Applicant: Oticon A/S, 2765 Smørum (DK)
(72) Inventor: Sommer, Peter, DK-2765 Smørum (DK); Bøgh, Carsten T., DK-2765 Smørum (DK); Madsen, Mads K., DK-2765 Smørum (DK); Madsen, Michael O., DK-2765 Smørum (DK)

(57) **Abstract**

A computer-implemented method for customizing a hearing aid, the hearing aid having one or more adjustable parameters, the method comprising: receiving trial period information comprising one or more of a performance parameter indicative of a performance of the hearing aid during a trial period, a usage parameter indicative of a usage pattern of the hearing aid during a trial period, user profile information indicative of one or more user characteristics, hearing aid information indicative of one or more hearing aid characteristics, and a user feedback parameter indicative of user information about a performance of the hearing aid during the trial period; determining one or more parameter settings of the one or more adjustable parameters based on the received trial period information; wherein determining comprises determining the one or more parameter settings based on the received trial period information, on previous trial period information from a plurality of previous trial periods performed for a corresponding plurality of other hearing aids and users, and on corresponding resulting parameter settings of respective adjustable parameters previously selected for said plurality of other hearing aids.

## Description

### TECHNICAL FIELD

The invention relates to the customization of a hearing aid.

### BACKGROUND

Hearing aids are generally customizable by setting one or more parameters of the hearing aid, thereby allowing adaptation of the hearing aid to specific needs of an individual user, a group of users, to specific environments, and/or the like. In particular, the hearing aid may need to be customized prior to normal use so as to suit the particular hearing deficiency of the user.

To this end, when a user is to use a new hearing aid, adjustable parameters of the hearing aid may be set during an initial customization process. This customization process is often performed by a hearing care professional such as an audiologist, normally as an interactive process between the hearing care professional and the user. Typically, the hearing care professional may select an initial setting of adjustable parameters of the hearing aid, e.g. a set of factory or default settings and/or a set of parameter settings that the hearing care professional may have selected based on information about the user and/or about the environment in which the hearing aid is to be used. The parameter settings selected by the hearing care professional during this initial fitting session will also be referred to as pre-trial parameter settings. After this initial fitting, the user may use the hearing aid during a certain trial period of time, e.g. a number of days, weeks or even months, so as to determine how well the pre-trial parameter settings accommodate the needs of the user. During this trial period, the hearing aid may store log data in a memory of the hearing aid. The log data may be indicative of a number of operational parameters, of usage patterns, and/or the like. The trial period allows the user to get accustomed to the new hearing aid and to try the hearing aid in a number of different situations and environments. After the trial period, the user is normally in a better position to provide feedback as to whether the selected pre-trial parameter settings are appropriate.

After the trial period, the hearing care professional may adjust the adjustable settings based on the user's experiences during the trial period and/or based on the log data.

To this end, the hearing care professional may use a fitting device such as a suitably programmed computer or other data processing system having an interface for connecting the hearing aid in a data communication connection to the data processing system. The fitting device may allow retrieval of log data from the memory of the hearing aid and/or the setting one of more of the adjustable parameters of the hearing aid.

European patent application EP2160923 discloses an interactive fitting process for hearing aids allowing the fitting of a hearing aid to suit the user's actual situations and the way the user uses his hearing aid, while taking into account the user's learning process when getting a new hearing aid and getting accustomed with it.

However, the customization process remains a time consuming task that requires considerable experience by the hearing care professional to select appropriate settings based on the user's feedback. Also, during the trial period, the hearing aid may not yet operate entirely satisfactorily, thus potentially causing frustration to the user or even dangerous situations. If the adjustment of the adjustable parameters after the trial period does not provide the desired improvement, the user may even have to return to the hearing care professional for one or even several additional adjustments. Moreover, many modern hearing aids have a large number of adjustable parameters, thus rendering the customization process a complicated task even for the experienced hearing care professional. Even though software exists for aiding the hearing care professional in the customization process, the process remains time consuming and complicated.

Accordingly, it is desirable to provide a more efficient and reliable customization process that increases the chances of selecting a suitable setting of the adjustable parameters based on few and/or short trial periods.

### SUMMARY

Disclosed herein are embodiments of a method for customizing a hearing aid, the hearing aid having one or more adjustable parameters, the method comprising
- receiving trial period information comprising one or more of a performance parameter indicative of a performance of the hearing aid during a trial period, a usage parameter indicative of a usage pattern of the hearing aid during a trial period, user profile information indicative of one or more user characteristics, hearing aid information indicative of one or more hearing aid characteristics, and a user feedback parameter indicative of user information about a performance of the hearing aid during the trial period;
- determining one or more parameter settings of the one or more adjustable parameters based on the received trial period information.

In embodiments of the method disclosed herein the one or more parameter settings are determined based on the received trial period information and on previous trial period information from a plurality of previous trial periods performed for a corresponding plurality of other hearing aids and other users, and on corresponding resulting parameter settings of respective adjustable parameters previously selected for said plurality of other hearing aids. The previous trial period information and corresponding resulting parameter settings may be stored on a data processing system implementing the method disclosed herein. Consequently, embodiments of the method disclosed herein allow information from previous customization processes to be used in subsequent customization processes, thus facilitating the selection of appropriate parameter settings and reducing the risk of having to perform repeated trial periods in order to arrive at appropriate parameter settings. Moreover, the stored trial period information and the stored resulting parameter settings may further be used by the hearing aid manufacturer during the development of future hearing aids and/or the optimization of factory settings of hearing aids.

Embodiments of the method disclosed herein may be performed automatically by a data processing system that automatically performs the setting of the adjustable parameters. Alternatively, the method may be implemented as an operator-assisted process where an operator of the data processing system, e.g. a hearing care professional, is presented with suggested parameter settings and provided with a user-interface for selecting other settings and/or for changing the suggested settings.

In some embodiments a system for customizing a hearing aid may comprise a fitting device and a central computer or other data processing system with which a plurality of fitting devices may communicate, e.g. via a suitable communications network such as the internet. The fitting device may be a special purpose apparatus or a suitably programmed conventional computer to which a hearing aid may be connectable, e.g. by a wired or wireless connection. The fitting device may provide functionality allowing an operator of the fitting device to enter information associated with a hearing aid such as user information or user feedback via a suitable user interface. The fitting device may further comprise functionality for receiving information from a hearing device connected to the fitting device, such as information about the type of instrument and/or one or more performance parameters and/or usage parameters. The fitting device may further be adapted to communicate with the central computer so as to send information entered by an operator of the fitting device and/or information received from a hearing aid.

The central computer may be adapted to maintain a repository of trial period information including information about a plurality of user profiles, associated information about hearing aids used by the respective users, performance, usage and/or feedback information. The repository may further comprise information related to the parameter settings of the adjustable parameters of the respective hearing devices during the trial periods and/or the parameter settings that resulted from the trial periods.

The repository may be stored in any suitable form such as a database, data warehouse, or the like. The central computer may be a server computer or other suitable data processing system. The central computer may be accessible via the internet or another suitable computer network.

The central computer may perform one or more data analysis steps on the information stored in the repository so as to detect patterns or features in the stored data and/or to perform a predictive analysis allowing the first computer to subsequently compute predictions of suitable parameter settings based on trial period information.

As new trial period information and associated parameter settings may be added to the repository during use of the system, the data analysis process may be repeated, e.g. periodically or triggered by certain events, e.g. operator input, a predetermined number or type of new trial period information having been added to the repository, and/or the like.

Upon receipt of new trial period information, the central computer may compute a predicted parameter setting based on the previously performed data analysis of the previous trial period information. The central computer may then send the computed predicted parameter settings as suggested parameter settings to the fitting device.

It will be appreciated that the central computer may be implemented as a single computer or as a plurality of computers. For example, one computer may maintain the repository and perform the data analysis resulting in a prediction function for predicting parameter settings from trial period information. Another computer may communicate with fitting devices and execute the prediction function.

Upon receipt of suggested parameter settings from the central computer, the fitting device may set the adjustable parameters of the hearing device connected to it. This may be done automatically or in an operator-assisted manner. For example, the fitting device may display the received suggested parameter settings and allow the operator to change some or all of the suggestions.

The determination of the parameter settings may comprise relating the received trial period information with the previous trial period information so as to determine a degree of similarity between the received trial period information and at least one of the previous trial period information; and determining the one or more parameters from said resulting parameter settings and from the determined degree of similarity. Consequently, the parameter settings determined by embodiments of the process disclosed herein may be chosen to be equal to parameter settings that have previously been chosen in a similar situation, e.g. for a user having similar user characteristics and/or in similar environments. The degree of similarity may be determined using any method known as such, e.g. based on a suitable metric. In some embodiments the parameter settings may be determined as a combination of a plurality of previously selected parameter settings, e.g. by averaging or otherwise interpolating between parameter settings previously determined for different users where the previous trial period information all have a certain degree of similarity to the presently received trial period information.

In some embodiments the method comprises performing a data analysis process so as to identify one or more patterns in the previous trial period information. Examples of such a data analysis process may comprise inspecting, cleaning, transforming, and modeling the data so as to extract relevant information that may be correlated to the parameter settings. To this end known techniques from data mining, machine learning, statistics, etc. may be used, e.g. clustering, supervised or unsupervised learning algorithms, and/or the like.

The received trial period information may comprise information available prior to the commencement of the trial period; this information will also be referred to as pre-trial information. Alternatively or additionally, the received trial period information may comprise information obtained during the trial period, i.e. information that is available only after completion of at least a part of the trial period; this information will also be referred to as post-trial information.

Similarly, the stored resulting parameter settings from previous fitting sessions relating to other hearing aids and/or users may comprise previous pre-trial parameter settings and/or previous post-trial parameter settings.

In some embodiments, the process comprises two stages: an initial stage in which the adjustable parameters are set to some initial pre-trial values based on information already available prior to the trial period, i.e. based only on pre-trial information. To this end, the hearing aid may be connected to a fitting device so as to allow an operator of the fitting device to set the adjustable parameters of the hearing aid to initial pre-trial values. The trial period is then performed with the parameters set to the determined pre-trial values. During this trial period, the user may use the hearing aid in various environments and situations, and the user may change some user-selectable parameters, e.g. selecting one of a number of programs, etc.

The setting of the pre-trial parameter settings may be performed by
- receiving a first subset of data available prior to the trial period, the first subset comprising at least one of user profile information and hearing aid information;
- determining one or more suggested pre-trial parameter settings of the one or more adjustable parameters based on the received first subset of data;
- receiving an operator input indicative of one or more adjusted pre-trial parameter settings;
- determining a measure of difference between the one or more suggested pre-trial parameter settings and the one or more adjusted pre-trial parameter settings; and
- using the determined measure during a subsequent determination of one or more suggested pre-trial parameter settings of another hearing aid.

Hence, the determination of the suggested pre-trial parameter settings by the data processing system based on pre-trial information may be improved over time, as the possible adjustments made by the hearing care professionals to the suggested pre-trial parameter settings are recorded and analyzed so as to determine a measure of difference between the initially suggested and subsequently adjusted pre-trial parameter settings. For example, the measure of difference may be an average difference, optionally weighted for different parameters, or weighted based on a measure of difference between the initially obtained pre-trial information. This analysis may thus result in a prediction of an average or typical adjustment made by the hearing care professionals to the suggested pre-trial parameter settings, e.g. as a function of the pre-trial information. This prediction may then be used to provide improved suggestions of pre-trial parameter settings during future fitting sessions.

During the trial period the hearing aid may collect post-trial information including data such as data indicative of usage patterns and performance data. Examples of such data may include information about when and how long each program has been selected, whether program shifts were automatic or manual, the number and pattern of volume adjustments, occurrences of feedback-caused howls, characteristics of the encountered listening environments, such as e.g. identified listening situations, signal levels and frequency distributions, noise levels and frequency distributions, etc.

After the trial period, the hearing aid may again be connected to a fitting device allowing the fitting device to retrieve usage and/or performance data from the hearing aid. Furthermore an operator of the fitting device may enter additional information e.g. based on user feedback about the trial period.

Based on the thus obtained trial period information, the system may determine suggested parameter settings as described herein, thus allowing the setting of the parameters to appropriate values in an efficient and reliable manner.

Accordingly, in some embodiments, the trial period information comprises
- a first subset of data available prior to the trial period, the first subset comprising at least one of user profile information and hearing aid information; and
- a second subset of data available only after completion of the trial period, the second subset comprising at least one of the performance parameter, the usage parameter and the user feedback parameter.

Some embodiments of the method comprise
- receiving the first subset of data;
- determining one or more pre-trial parameter settings of the one or more adjustable parameters based on the received trial period information;
- receiving the second subset of data after completion of a trial period with the adjustable parameters set to the determined pre-trial parameter settings;
- determining one or more post-trial parameter settings of the one or more adjustable parameters based on the received first and second subsets of data;
wherein determining the one or more pre-trial parameter settings and determining the one or more post-trial parameter settings are each based on previous trial period information from a plurality of previous trial periods performed for a corresponding plurality of other hearing aids and users, and on corresponding resulting parameter settings of respective adjustable parameters previously selected for said plurality of other hearing aids. In a more general embodiment, at least determining the one or more post-trial parameter settings is based on previous trial period information from a plurality of previous trial periods performed for a corresponding plurality of other hearing aids and users, and on corresponding resulting parameter settings of respective adjustable parameters previously selected for said plurality of other hearing aids.

Generally, the user profile information indicative of one or more user characteristics may include one or more of the following: The user's age, the user's gender, information about the user's lifestyle (e.g. by assigning one or more life-style attributes such as active, inactive, etc.), information about the user's hearing deficiency (e.g. an audiogram), information about which types of acoustic environments the user is likely to experience or is particularly concerned about (e.g. traffic, music, etc.), and/or the like.

The hearing aid information indicative of one or more hearing aid characteristics may include a type of hearing aid, information identifying the hearing aid program(s) stored in the hearing aid, information as to whether the hearing aid is monaural or binaural, information whether the hearing aid is connectable to other devices, etc.

The above user profile information and hearing aid information are normally available prior to the trial period, and may e.g. be entered into the fitting device during an initial visit of the hearing aid user at the hearing care professional.

The performance parameter indicative of a performance of the hearing aid during a trial period may include statistical data about the sound pressure, noise level and/or other detectable parameters that indicate the acoustic environment in which the hearing aid has operated.

The usage parameter indicative of a usage pattern of the hearing aid during a trial period may include statistical data indicative of the overall usage patterns, e.g. the total number of hours of operation, the number of hours of operation in each of a number of user-selectable programs, the number of hours of operation during different times of day, etc. Alternatively or additionally, the usage parameter may include statistical data indicative of settings and/or user choices during the trial period, e.g. volume control settings, etc.

The user feedback parameter indicative of user information about a performance of the hearing aid during the trial period may include information provided by the user about the perceived performance of the hearing aid. For example, the information may be entered in the form of a computerized questionnaire. Alternatively, or additionally, the information may be derived from the data indicative of usage patterns and performance data.

In some embodiments the hearing aid may communicate with the central computer via a different communications channel not involving the fitting device of a hearing care professional. For example, the hearing aid may be in communicative connection with a gateway device that provides connectivity to a computer network, e.g. wireless connectivity via a short range radio-frequency communications technology such as a wireless local area network (WLAN), Bluetooth, or the like. The connection between the hearing aid and the gateway device may e.g. be provided by an inductive connection, a wired connection, or in any other suitable way known as such in the art. Hence, the hearing aid may communicate, via the gateway device and a suitable computer network e.g. including the internet with the central computer. Consequently, the hearing aid may communicate its parameter settings to the central computer, and receive updated (pre- or post-trial) parameter settings from the central computer. In such an embodiment, a computer of the computer network thus plays the role of the fitting device. This may e.g. be a personal computer of the user of the hearing aid, e.g. a personal computer to which the gateway device is connectable via a short-range wireless connection. The user of the hearing aid may e.g. use the personal computer to visit a website that allows uploading of hearing aid parameters and/or the downloading of suggested updated (pre- or post-trial) parameters to the hearing aid. The personal computer may be a stationary computer or a portable computer or computing device, e.g. a tablet computer or a smartphone.

The features of embodiments of the method described herein may be implemented in software and carried out on a data processing system caused by the execution of computer-executable instructions. The instructions may be program code means loaded in a memory, such as a RAM, from a storage medium or from another computer via a computer network. Alternatively, the described features may be implemented by hardwired circuitry instead of software or in combination with software.

The present invention relates to different aspects including the method described above and in the following, corresponding methods, devices, and/or product means, each yielding one or more of the benefits and advantages described in connection with the first mentioned aspect, and each having one or more embodiments corresponding to the embodiments described in connection with the first mentioned aspect and/or disclosed in the appended claims.

### BRIEF DESCRIPTION OF THE DRAWINGS

Aspects of the method and system for customizing a hearing aid disclosed herein will be described in more detail in the following description of preferred embodiments and with reference to the drawings.
Fig. 1 illustrates an example of a system for customizing a hearing aid.
Fig. 2 illustrates another example of a system for customizing a hearing aid.
Fig. 3 shows an example of a customization process.

Throughout the drawings like reference numerals are used to refer to the same or like components, features or elements.

### DETAILED DESCRIPTION

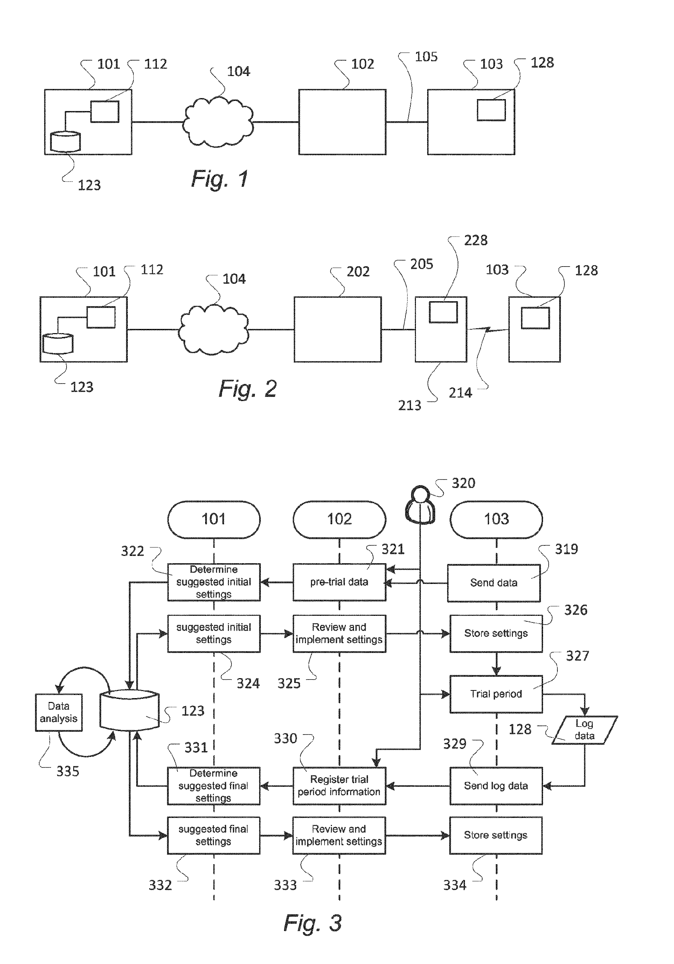

Fig. 1 illustrates an example of a system for customizing a hearing aid. The system comprises a hearing aid 103, a fitting device 102 and a central data processing system 101. The hearing aid 103 is connectable to the fitting device, e.g. via a wired connection 105. The fitting device 102 may be a suitably programmed general-purpose computer or a special-purpose device. The fitting device has an interface allowing a hearing aid to be connected to it. The fitting device is further connectable to the central data processing system 101, e.g. via a suitable computer network 104 such as the internet. The fitting device has further input means, e.g. a suitable user-interface allowing entering of information related to the hearing aid or to the user of the hearing aid. The system is configured to facilitate the customization process described herein. In particular, when the hearing aid is to be customized to a user, the hearing aid may be connected to the fitting device via connection 105. An operator of the fitting device may enter data related to the user of the hearing aid and/or data related to the hearing aid into the fitting device. Furthermore, the fitting device may receive data directly from the hearing aid via connection 105, e.g. information about the type of hearing aid, current settings of the hearing aid, and/or the like.

The fitting device is further in communicative connection with the central data processing system 101, e.g. via the internet or another suitable computer network 104, thus allowing the communication of data related to the hearing aid and/or the user of the hearing aid from the fitting device to the central data processing system 101 before and/or after a trial period.

In particular, when the hearing aid is connected to the fitting device prior to a trial period, data about the hearing aid, about the user, and about expected usage patterns of the hearing aid by the user may be entered into the fitting device. Some or all of the data may then be forwarded to the central data processing system. The hearing aid comprises a memory 128 or other storage device for storing performance data and/or usage related data. Accordingly, when the hearing aid is connected to the fitting device after completion of the trial period, the fitting device may further receive performance and usage related data from the hearing aid as well as feedback information received from the user and entered by the operator of the fitting device.

The fitting device may further receive data from the central data processing system via computer network 104. In particular, the central data processing system may send suggested hearing aid settings responsive to the information received by the central data processing system from the fitting device.

To this end, the central data processing system comprises a database 123 or similar repository. The database 123 has stored thereon trial period information about a plurality of hearing aids, hearing aid users, usage patterns, parameter settings, performance data, user feedback information, etc. that have previously been received by the central data processing system. In particular, the database may have stored thereon trial period information related to a plurality of users, hearing aids, usage patterns etc. and the hearing aid settings that were ultimately selected based on these trial periods. Consequently, the database comprises a large knowledge base for associating trial period information with parameter settings.

The central data processing system further comprises a data analysis module 112 configured to perform data analysis of the data stored in the database 123. The data analysis module may perform one or more data analysis steps on the information stored in the database 123 so as to detect patterns or features in the stored data and/or to perform a predictive analysis allowing the first computer to subsequently compute predictions of suitable parameter settings based on trial period information. For example, the data analysis module may perform a statistical analysis of the stored information, a machine learning process, and/or another suitable data analysis process. The data analysis process may further perform data cleansing, data compression and/or similar data analysis steps, e.g. for detecting outliers or otherwise atypical or unreliable data.

In some embodiments, the data analysis module may perform a supervised learning process based on the previously received data so as to predict a functional relationship between the trial period information and the resulting parameter settings. The resulting functional relationship may then be used as a predictor for estimating suitable parameter settings responsive to future trial period information. The data may e.g. be analysed so as to determine the average or typical change - as a function of the trial period information - between the resulting parameter settings and the settings initially suggested by the fitting system. The data analysis process may be executed repeatedly, e.g. upon receipt of new trial period information and related parameter settings, so as to continuously improve the resulting predictors.

Based on the data analysis and upon receipt of new trial period information, the central data processing system computes predicted parameter settings based on the previously performed data analysis of the previous trial period information. In one embodiment, the central data processing system may compute a distance measure comparing the received trial period information with each of a plurality of previously received information, so as to determine a degree of similarity between the current trial period information and the previously received trial period information. Such distance measures are well-known in the art as such. Based on the distance measure, the central data processing system may determine one or more previous trial period information that were most similar to the current trial period information. Based on this determination, the central data processing system may then estimate suggested parameter settings to be equal to the previous parameter settings that resulted from the most similar previous trial period information, or as a suitable interpolation of multiple parameter settings from a set of similar previous trial period information.

The central data processing system computer sends the computed predicted parameter settings as suggested parameter settings to the fitting device. The fitting device may in turn display the received suggested parameter settings and even allow the operator of the fitting device to adjust the parameters, e.g. based on the operator's own experience. The fitting device then, via the connection 105, performs the setting of the hearing aid's parameters to the final parameter settings. The fitting device may further return the final parameter settings to the central data processing system so as to allow the central data processing system to include them in the database together with the corresponding trial period information.

It will be appreciated that the information available at the fitting device may vary depending on whether a trial period has already been performed, how much data and/or feedback have been received from the trial period, etc. Consequently, the resulting suggested parameter settings may correspond to more or less accurate estimates of the most appropriate parameter settings. For example, when the hearing aid is connected to the fitting device during an initial session prior to the trial period, the central data processing system may return a preliminary suggestion of parameter settings based on the information about the hearing aid and the user available at that time. When the hearing aid is again connected to the fitting device after completion of the trial period, the fitting device may retrieve performance data and/or usage data from a memory of the hearing aid, and the operator may enter feedback information received from the user of the hearing aid. Based on this supplementary information, the central data processing system then provides an updated suggestion of suitable parameters settings.

Fig. 2 illustrates another example of a system for customizing a hearing aid. The system of fig. 2 is similar to the system of fig. 1 in that it comprises a hearing aid 103, a local computer 202, and a central data processing system 101. The local computer 202 may e.g. be a personal computer or other computing device of the user of the hearing aid 103.

As in the previous example, the local computer 202 is connectable to the central data processing system, thus allowing the exchange of trial period information and hearing aid parameter setting via a suitable computer network 104, e.g. the internet. The central data processing system 101 comprises a database 123 and a data analysis module 112, and the central data processing system is adapted to maintain a repository of trial period information and corresponding parameter settings in the data base 123, to perform a suitable data analysis of the stored data, to receive trial period information from the local computer 202, and to return corresponding suggested parameter settings to the local computer 202, all as described with reference to the central data processing system 101 and the fitting device 102 of fig. 1.

In the example of fig. 2, the hearing aid is connected to the local computer via a gateway device 213, e.g. a gateway device worn or carried by the user of the hearing aid. The gateway device may be in communicative connection 214 with the hearing aid, e.g. via an inductive coupling, a short-range radio-frequency connection or in another suitable way. The gateway device 213 is in turn connectable to the local computer 202 via a suitable data connection 205, e.g. a wired or wireless connection, for example a short-range radio-frequency connection using a suitable communications protocol. For example the gateway device 213 may be connected to the local computer via a Bluetooth connection.

Even though the hearing aid may comprise a memory 128 for storing log data as described above, the gateway device 213 of fig. 2 may comprise a memory 228 for storing log data in addition or alternative to the log data stored by the hearing aid. Hence, in the example of fig. 2, performance data and usage data may be stored in the gateway device and transmitted to the local computer 202. As the gateway device may not necessarily be subject to the same constraints as regards size and power consumption as the hearing aid, the memory of the gateway device may be larger thus allowing the collection of more detailed log data. Furthermore, the gateway device may be adapted to forward log data not only at the end of the trial period by even during the trial period, e.g. every time the gateway is in proximity of the local computer 202.

This allows the collection of an increased amount of data and thus of more detailed log data and an improved prediction of suitable parameter settings. Furthermore, a more frequent communication with the local computer allows the local computer to query the central data processing system more frequently about suggested parameter settings, thus allowing a continuous improvement of the parameter settings or a detection of inappropriate settings.

As mentioned above, the local computer 202 may be the user's own computer. The local computer may execute suitable software for retrieving trial period information from the gateway device and to forward the collected information to the central data processing system. To this end, the user may use the local computer 202 to visit a website allowing the user to enter information such as user information, trial period feedback, etc. and/or to upload data retrieved from the gateway device 213. Alternatively, the uploading of information to the central data processing system may be performed automatically, e.g. by a suitable service running on the local computer 202. This service may then alert the user when there are updated parameter settings available. The service may also alert the user so as to suggest the user to visit a hearing care professional or reminding the user about an appointment with the hearing care professional.

In the following, an example of a customization process will be described with reference to fig. 3. The process will be described with further reference to the system of fig. 1. However, it will be appreciated that corresponding embodiments of the process may be performed by the system of fig. 2 or similar systems.

The process is initiated by a hearing aid 103 being connected to the fitting device 102, and the hearing aid sending information specific to the hearing aid to the fitting device (step 319). Examples of such data include data indicative of the type and/or version of the hearing aid, current parameter settings, other characteristics of the hearing aid, etc. In step 321, the fitting device receives the data from the hearing aid as well as user-specific data including information about the user 320 of the hearing aid. The user-specific data may be entered into the fitting device by an operator of the fitting device, e.g. by a hearing care professional. Additionally or alternatively, some or all of the user-specific data may be received by the fitting device via a suitable data interface, e.g. from another computer, from a data storage device, and/or the like. Examples of user-specific data include the user's age and gender, information about the user's lifestyle, information about the user's hearing deficiency (e.g. an audiogram), information about which types of acoustic environments the user is likely to experience or is particularly concerned about (e.g. traffic, music, etc.), and/or the like. The fitting device forwards the received information, or at least a subset of the received information, to the central data processing system 101.

In step 322, the central data processing system determines suggested pre-trial parameter settings for the hearing aid. To this end, the central data processing system performs a data analysis process on previously received data that is stored in database 123 and related to a plurality of hearing aids and users, as described herein.

For example, the central data processing system 101 may perform a data analysis process 335 for analyzing the data stored in the database 123 and resulting in suitable predictor functions associating input data about a hearing aid and a user profile to suggested parameter settings for the hearing aid. The predictor functions may be determined from data from previous fitting sessions, in particular from previous pre-trial information, the resulting suggested pre-trial parameter settings and from previous pre-trial parameter settings as subsequently adjusted by a hearing aid professional and/or from the ultimately determined post-trial parameter settings after previous trial periods. The data analysis process may thus result in a model that is used in step 322 to determine suggested pre-trial parameter settings for the current hearing aid and the current user. The parameter settings may include e.g. amplification settings for respective frequency ranges, knee-points for level compression, aggressiveness of noise reduction and/or feedback suppression, speed of automatic program changes, the inclusion or removal of certain programs for different acoustic environments or usage patterns, and/or the like.

In step 324, the central data processing system forwards the suggested pre-trial parameter settings to the fitting device 102, where they may be displayed and where the operator of the fitting device may be allowed to change some or all of the suggested parameters (step 325). Once the suggested or amended settings are accepted by the operator of the fitting device, the fitting device forwards the pre-trial parameter settings to the hearing aid where they are stored/applied (step 326).

After the above initial customization stage, the hearing aid is disconnected from the fitting device and ready for use by the user during a trial period. It will be appreciated that in some embodiments, the setting of the pre-trial parameter values may be performed without involving the central data processing system. For example, the fitting device may have stored thereon a number of standard pre-trial parameter settings for different types of users/hearing aids, and/or the operator of the fitting device may manually select some or all of the pre-trial parameter settings based on his/her experience.

In any event, after the parameters of the hearing aid have been set to their pre-trial values, the user uses the hearing aid during a trial period 327, e.g. for one or a few weeks, with the hearing aid parameters set to the pre-trial parameter settings. During this trial period, the hearing aid may store log data 128 in its internal memory, the log data being indicative of one or more performance or usage statistics, such as statistical data about the sound pressure, noise level and/or other detectable parameters that indicate the acoustic environment in which the hearing aid has operated. The log data may further include usage data indicative of a usage pattern of the hearing aid during a trial period, e.g. statistical data indicative of the overall usage patterns, such as the total number of hours of operation, the number of hours of operation in each of a number of user-selectable programs, the number of hours of operation during different times of day, etc. Alternatively or additionally, the usage data may include statistical data indicative of settings and/or user choices during the trial period, e.g. volume control settings, etc.

After completion of the trial period, the hearing aid is again connected to the fitting device 103, and the hearing aid forwards the log data acquired during the trial period to the fitting device (step 329). The fitting device further receives feedback information from the user 320 about the perceived appropriateness of the parameter settings (step 330). For example, the feedback information may be entered by way of a standardized questionnaire (e.g. a multiple choice questionnaire) allowing subsequent automatized data analysis of the received answers. The fitting device forwards the received information, or at least a subset of the received information, to the central data processing system 101.

In step 331, the central data processing system determines suggested settings for the hearing aid. To this end, the central data processing system predicts suitable settings based on a data analysis of previously stored trial period information in the database 123. This prediction process is similar to the process described in connection with step 322, but based on additional input data, namely the log data and user feedback data, thus allowing a more accurate determination of suggested settings.

In step 332, the central data processing system forwards the suggested parameter settings to the fitting device 102, where they may be displayed and where the operator of the fitting device may be allowed to change some or all of the suggested parameters (step 333). Once the suggested or amended settings are accepted by the operator of the fitting device, the fitting device forwards the settings to the hearing aid where they are stored/applied (step 334). The fitting device may further send the final settings to the central data processing system where they are stored in the database 123 together with the corresponding trial period results, user data, and hearing aid data for use by the data analysis process 335 and future predictions of suggested settings.

After the above second customization stage, the hearing aid is disconnected from the fitting device and ready for use by the user.

Although some embodiments have been described and shown in detail, the invention is not restricted to them, but may also be embodied in other ways within the scope of the subject matter defined in the following claims.

The method, product means, system, and device described herein can be implemented by means of hardware comprising several distinct elements, and/or partly or completely by means of a suitably programmed microprocessor. In the device claims enumerating several means, several of these means can be embodied by one and the same item of hardware, e.g. a suitably programmed microprocessor, one or more digital signal processor, or the like. The mere fact that certain measures are recited in mutually different dependent claims or described in different embodiments does not indicate that a combination of these measures cannot be used to advantage.

It should be emphasized that the term "comprises/comprising" when used in this specification is taken to specify the presence of stated features, integers, steps or components but does not preclude the presence or addition of one or more other features, integers, steps, components or groups thereof.

## Claims

1. A computer-implemented method for customizing a hearing aid, the hearing aid having one or more adjustable parameters, the method comprising
- receiving trial period information comprising one or more of a performance parameter indicative of a performance of the hearing aid during a trial period, a usage parameter indicative of a usage pattern of the hearing aid during a trial period, user profile information indicative of one or more user characteristics, hearing aid information indicative of one or more hearing aid characteristics, and a user feedback parameter indicative of user information about a performance of the hearing aid during the trial period;
- determining one or more parameter settings of the one or more
adjustable parameters based on the received trial period information;
wherein determining comprises determining the one or more parameter settings based on the received trial period information, on previous trial period information from a plurality of previous trial periods performed for a corresponding plurality of other hearing aids and users, and on corresponding resulting parameter settings of respective adjustable parameters previously selected for said plurality of other hearing aids.

2. A method according to claim 1; wherein determining comprises
- relating the received trial period information with the previous trial period information so as to determine a degree of similarity between the received trial period information and at least one of the previous trial period information; and
- determining the one or more parameters from said resulting parameter settings and from the determined degree of similarity.

3. A method according to claim 1 or 2, comprising performing a data analysis process so as to identify one or more patterns in the previous trial period information, and wherein determining comprises determining the one or more parameter settings based on at least the retrieved trial period information and on the identified patterns.

4. A method according to any one of the preceding claims, wherein the trial period information comprises
- a first subset of data available prior to the trial period, the first subset comprising at least one of user profile information and hearing aid information; and

5. A method according to claim 4, wherein the method comprises
- receiving the first subset of data;
- determining one or more suggested pre-trial parameter settings of the one or more adjustable parameters based on the received first subset of data;
- receiving an operator input indicative of one or more adjusted pre-trial parameter settings;
- determining a measure of difference between the one or more suggested pre-trial parameter settings and the one or more adjusted pre-trial parameter settings; and
- using the determined measure during a subsequent determination of one or more suggested pre-trial parameter settings of another hearing aid.

6. A method according to claim 4 or 5, wherein the trial period information further comprises a second subset of data available only after completion of the trial period, the second subset comprising at least one of the performance parameter the usage parameter and the user feedback parameter.

7. A method according to claim 6, comprising
- receiving the first subset of data;
- determining one or more pre-trial parameter settings of the one or more adjustable parameters based on the received trial period information;
- receiving the second subset of data after completion of a trial period with the adjustable parameters set to the determined pre-trial parameter settings;
- determining one or more post-trial parameter settings of the one or more adjustable parameters based on the received first and second subsets of data;
wherein determining the one or more pre-trial parameter settings and determining the one or more post-trial parameter settings are each based on previous trial period information from a plurality of previous trial periods performed for a corresponding plurality of other hearing aids and users, and on corresponding resulting parameter settings of respective adjustable parameters previously selected for said plurality of other hearing aids.

8. A method according to any one of the preceding claims, comprising including the received trial period information and the determined parameter settings in the plurality of previous trial period information.

9. A data processing system having stored thereon computer program means adapted to cause the data processing system to perform the steps of the method of any one of claims 1 through 8 when the computer program means are executed by the data processing system.

10. A data processing system according to claim 9, comprising a first computer having stored thereon said computer program means adapted to cause the first computer to perform the steps of the method of any one of claims 1 through 8 when the computer program means are executed by the first computer; and a fitting device comprising a first interface for connecting the hearing aid to the fitting device to allow data communication between the fitting device and the hearing aid; and a second interface for providing data communication between the fitting device and the computer; wherein the fitting device is adapted to
- receive at least one of the usage parameter and the performance parameter from the hearing aid via the first interface;
- to send the trial period information to the first computer via the second interface;
- to receive the determined parameter settings from the first computer via the second interface; and
- to cause, via the first interface, setting of the adjustable parameters of the hearing aid to the determined parameter settings.

11. A computer program product comprising computer program means adapted to cause a data processing system to perform the steps of the method of any one of claims 1 through 8 when the computer program means are executed by the data processing system.
